# EUROPÄISCHE PATENTSCHRIFT

(11) **EP 1 627 650 B1**
(45) Veröffentlichungstag und Bekanntmachung des Hinweises auf die Patenterteilung: **25.03.2009**
(21) Anmeldenummer: 05017470.5
(22) Anmeldetag: 11.08.2005
(51) Int. Cl.: A61M 1/16, A61J 1/05

(54) **Bicarbonatbehälter mit Zweikanal-Steckverbinder zum Einmalgebrauch in Hämodialysegeräten**
Bicarbonate container with double-channel connector for single use in haemodialysis apparatuses
Container à bicarbonate avec connecteur à double canal pour usage unique dans des dispositifs d'hémodialyse

(30) Priorität: 18.08.2004 DE 102004039989
(43) Veröffentlichungstag der Anmeldung: 22.02.2006
(73) Patentinhaber: Ritter GmbH, 86830 Schwabmünchen (DE)
(72) Erfinder: Ritter, Frank, 87745 Eppishausen (DE)
(74) Vertreter: Gallo, Wolfgang

(56) Entgegenhaltungen:
- EP-A1- 0 475 825
- FR-A- 2 749 763

## Beschreibung

Die Erfindung betrifft Bicarbonatbehälter zum Einmalgebrauch in Dialysegeräten zur Blutwäsche, und zwar einen solchen Bicarbonatbehälter in der Ausführung als steifen Kunststoffbehälter und mit zweikanaligem Steckanschluß zur Verbindung mit dem Dialysegerät.

Ein Bicarbonatbehälter mit zweikanaligem Steckverbinder ist bereits aus der EP-0 575 970 A2 bekannt und ist dort als flexibler Beutel ausgebildet. Da wegen des doppelkanaligen Steckverbinders zum Anschluß an das Dialysegerät Einlaß und Auslaß für den Lösungskreislauf an derselben Stelle des Behälters liegen, muß innerhalb des Behälters durch einen von einem der beiden Steckverbinderkanäle zum entgegengesetzten Behälterende führenden Leitungskanal dafür gesorgt werden, dass der gesamte Bicarbonatbehälter von der Lösungsflüssigkeit durchströmt werden kann. Dies geschieht bei dem bekannten Behälter dadurch, dass von einem der beiden Steckverbinderkanäle aus ein flexibler Schlauch in das Beutelinnere hineinragt und etwa an der Steckverbindung gegenüberliegenden Beutelende ausmündet.

Abgesehen davon, dass diese Anordnung einen relativ hohen Fertigungsaufwand erfordert, besteht auch die Gefahr, dass das freie Ende des in das Beutelinnere hineinragenden Schlauchs in dem mit Bicarbonat gefüllten Beutel nicht oder nicht mehr die vorgesehene Lage hat und dann der Behälter tatsächlich nur teilweise von der Lösungsflüssigkeit durchströmt wird.

Der Erfindung liegt die Aufgabe zugrunde, einen insbesondere hinsichtlich wesentlich vereinfachter Fertigung und sicher definierter Position der Ausmündung eines von der Steckverbindung zum gegenüberliegenden Behälterende führenden Leitungskanals verbesserten Bicarbonatbehälter zu schaffen.

Diese Aufgabe wird gemäß der Erfindung durch den im Anspruch 1 angegebenen Bicarbonatbehälter gelöst. Vorteilhafte Ausgestaltungen der Erfindung sind Gegenstand der Unteransprüche.

Der erfindungsgemäße Bicarbonatbehälter ist als steifer Kunststoffbehälter ausgebildet und besteht aus einem Basisteil mit aufgeschweißtem Deckel, wobei der von der Steckverbindung zum entgegengesetzten Behälterende verlaufende Leitungskanal zwischen dem Basisteil und dem Deckel angeordnet ist. Auf diese Weise ist eine einfachen Fertigung des Behälters möglich, indem auf das Basisteil, wie es aus der Spritzgussmaschine kommt, nur noch der Deckel aufgeschweißt zu werden braucht.

Die Erfindung wird nachstehend anhand zweier Ausführungsbeispiele unter Bezugnahme auf die anliegenden Zeichnungen mehr im einzelnen beschrieben. In den Zeichnungen zeigt:
- Fig. 1: Eine Draufsicht auf das Basisteil einer ersten Ausführungsform des Bicarbonatbehälters nach der Erfindung,
- Fig. 2: einen Schnitt durch das Basisteil längs der Linie II-II in Fig. 1,
- Fig. 3: eine Untersicht des Deckelteils des Bicarbonatbehälters der ersten Ausführungsform,
- Fig. 4: einen Schnitt durch das Deckelteil längs der Linie IV-IV in Fig. 3,
- Fig. 5: eine Draufsicht auf den vollständigen Bicarbonatbehälter der ersten Ausführungsform, wobei die Schweißflächen zwischen Basisteil und Deckelteil gestrichelt schraffiert sind,
- Fig. 6: eine Draufsicht auf eine zweite Ausführungsform des Bicarbonatbehälters nach der Erfindung,
- Fig. 7: eine Untersicht des Bicarbonatbehälters nach Fig. 6,
- Fig. 8: einen Längsschnitt durch den Bicarbonatbehälter der zweiten Ausführungsform längs der Linie VIII-VIII in Fig. 7,
- Fig. 9: in vergrößerter Darstellung die Einzelheit IX in Fig. 8 und
- Fig. 10: in vergrößerter Darstellung die Einzelheit X in Fig. 8.

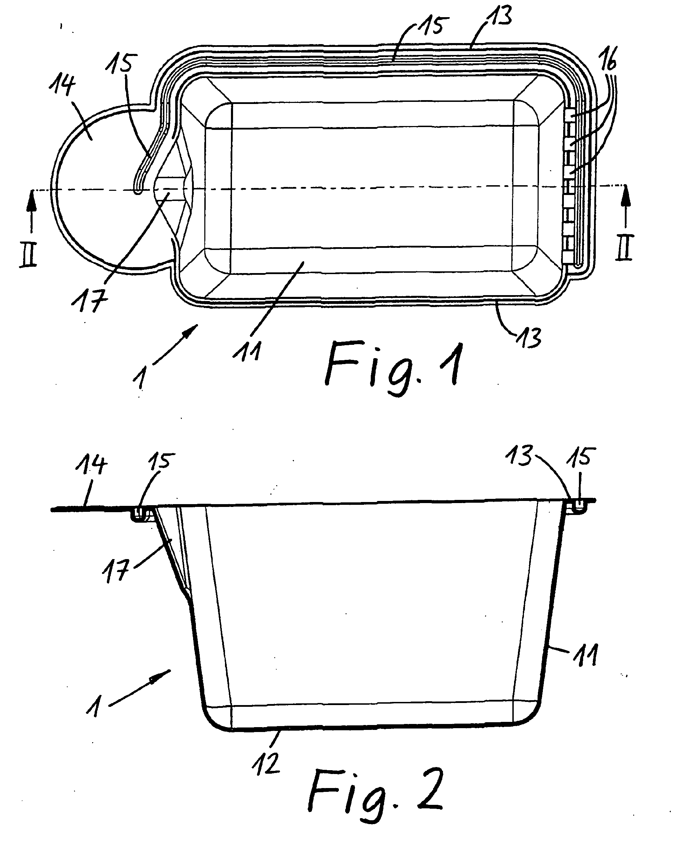

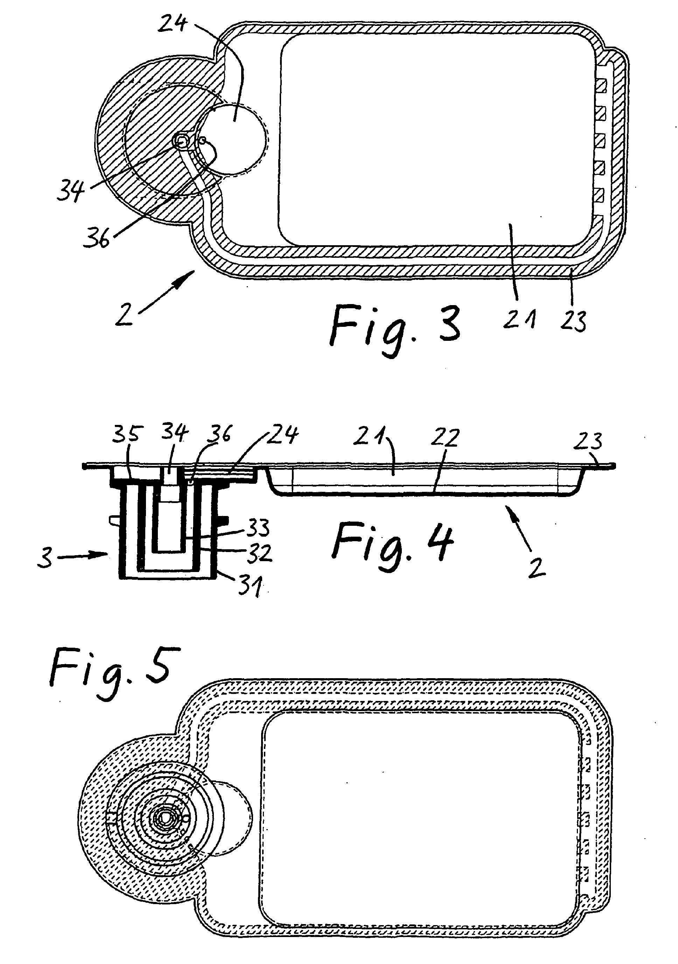

Der dargestellte Bicarbonatbehälter in den Fig. 1 bis 5 der ersten Ausführungsform besteht aus einem Basisteil 1 (Fig. 1 und 2) und einem darauf aufgesetzten und damit verschweißten Decktelteil 2 (Fig. 3 und 4). Beide Teile sind im Spritzgussverfahren aus Kunststoff hergestellt.

Das Basisteil 1 ist als Wanne 11 mit einem Boden 12 und einem oberen Flansch 13 ausgebildet. Die Wanne 11 hat beim dargestellten Ausführungsbeispiel eine im wesentlichen rechteckige Form mit gerundeten Ecken und einer leichten Verjüngung zum Boden 12 hin, aber die Wanne könnte auch eine beliebige andere Form wie beispielsweise rund oder halbkugelig oder dergleichen haben. Der Flansch 13 ist flach und verläuft in der Öffnungsebene der Wanne 11 um die Wannenöffnung herum. Die Oberseite des Flanschs 13 bildet eine Schweißfläche zur Verschweißung mit einer entsprechenden Flanschfläche des Deckelteils 2.

Das Deckelteil 2 ist gemäß den Fig. 3 und 4 als flache Wanne 21 mit einem Boden 22 und einem die Wannenöffnung in der Öffnungsebene umschließenden ebenen Flansch 23 ausgebildet. An einer Schmalseite der Wanne 21 ist der Flansch 23 vergrößert und mit einem angeformten Steckverbinder 3 ausgebildet, der, wie die Schnittdarstellung nach Fig. 4 zeigt, eine äußere Führungshülse 31 sowie innerhalb derselben und damit konzentrisch angeordnet zwei koaxiale Leitungsbuchsen 32 und 33 aufweist. Die äußere Führungshülse 31 dient zur Führung und mechanischen Fixierung sowie Verriegelung des Bicarbonarbehälters an der entsprechend komplementär ausgebildeten Steckverbinderaufnahme des Dialysegeräts. Die äußere Leitungsbuchse 32 und die innere Leitungsbuchse 33 bilden miteinander zwei koaxiale Leitungskanäle, die als Einlaß und Auslaß für die Lösungsflüssigkeit dienen. Der von der inneren Leitungsbuchse 33 begrenzte innere Leitungskanal endigt in der Flanschebene in einer zentrischen Öffnung 34, und der zwischen der inneren Leitungsbuchse 33 und der äußeren Leitungsbuchse 32 gebildete ringförmige Leitungskanal ist duch einen Boden 35 abgeschlossen, der eine Durchtrittsöffnung 36 aufweist, die in eine flache kreisförmige Vertiefung 24 des Flanschs 23 ausmündet.

Der Flansch 13 des Basisteils 1 weist gemäß den Fig. 1 und 2 an einer Schmalseite der Wanne 11 eine kreissegmentförmige Erweiterung 14 auf, die komplementär zu dem mit dem Steckverbinder 3 ausgebildeten Teil des Flanschs 23 des Deckelteils 1 ausgebildet ist. Außerdem ist im Flansch 13 des Basisteils 1 eine vom Zentrum der Flanscherweiterung 14 ausgehende und längs einer Längsseite der Wanne um die Wannenöffnung herumgeführte und zu der der Flanscherweiterung 14 gegenüberliegenden Schmalseite der Wanne verlaufende Rinne 15 gebildet, die jedoch durch einen zwischen ihr und der Wannenöffnung verlaufenden streifenförmigen Flanschbereich von der Wannenöffnung getrennt ist. Diese Rinne verläuft im wesentlichen über die ganze Breite der der Flanscherweiterung 14 gegenüberliegenden Schmalseite der Wanne 11 und steht dort über eine Reihe von im Flansch gebildeten Übertrittsöffnungen 16 mit der Wannenöffnung in Verbindung.

Wie weiter aus den Fig. 1 und 2 hervorgeht, hat die Wanne 11 an ihrer der Flanscherweiterung 14 entsprechenden Schmalseite zur Wannenöffnung hin eine Ausbauchung 17.

Fig. 5 zeigt eine Draufsicht des durch Aufsetzen des Deckelteils 2 auf das Basisteil 1 und Verschweißen von Deckelteil und Basisteil an den aneinander anliegenden Flanschflächen fertiggestellten Bicarbonatbehälters nach der Erfindung.

Der Flächenbereich, über welchen der Flansch 13 des Basisteils mit dem Flansch 23 des Deckelteils verschweißt ist, ist in Fig. 5 durch eine gestrichelte Schraffierung markiert. Die entsprechenden Schweißflächen am Deckelteil sind auch in Fig. 3 durch eine Schraffur kenntlich gemacht. Daraus erkennt man, dass beim Flansch 23 des Deckelteils 2 der der Rinne 15 im Flansch 13 des Basisteils 1 entsprechende Streifenbereich ausgespart ist.

Aus Fig. 5 ist nun, insbesondere in der Zusammenschau mit den anderen Zeichnungsfiguren, ersichtlich, dass nach dem Verschweißen der Flansche 13 und 23 von Basisteil 1 und Deckelteil 2 die Rinne 15 im Basisteilflansch 13 an ihrer ursprünglich offenen Oberseite nun durch den Deckelteilflansch 23 verschlossen ist und damit einen Leitungskanal bildet, der vom Steckverbinder 3 ausgehend von dem durch die Wannen 11 und 21 von Basisteil und Deckelteil gebildeten Innenraum getrennt zu dem vom Steckverbinder 3 entfernt gelegenen Ende des Behälters geführt ist und erst dort über die vom Steckverbinder 3 entfernte Schmalseite durch die Übertrittsöffnungen 16 mit dem Behälterinneren in Verbindung steht. Im Bereich des Steckverbinders 3 steht dieser durch die Rinne 15 gebildete Leitungskanal mit der Öffnung 34 und somit mit dem von der inneren Leitungsbuchse 33 umschlossenen Leitungskanal in Verbindung. Die über die Öffnung 36 im Boden 35 mit dem ringförmigen Leitungskanal zwischen der inneren Leitungsbuchse 33 und der äußeren Leitungsbuchse 32 in Verbindung stehende kreisförmige Vertiefung 24 des Deckelteilflanschs 23 befindet sich beim fertigen Bicarbonatbehälter über dem entsprechenden Endbereich der Wanne 11 einschließlich der Ausbauchung 17.

Auf diese Weise ist also der eine, nämlich der ringförmige Leitungskanal des Steckverbinders 3, mit dem steckverbinderseitigen Ende des Behälterinnenraums verbunden, während der innere Leitungskanal des Steckverbinders 3 über den durch die Rinne 15 gebildeten Leitungskanal und die Übertrittsöffnungen 16 mit dem entgegengesetzten Ende des Bicarbonatbehälters verbunden ist.

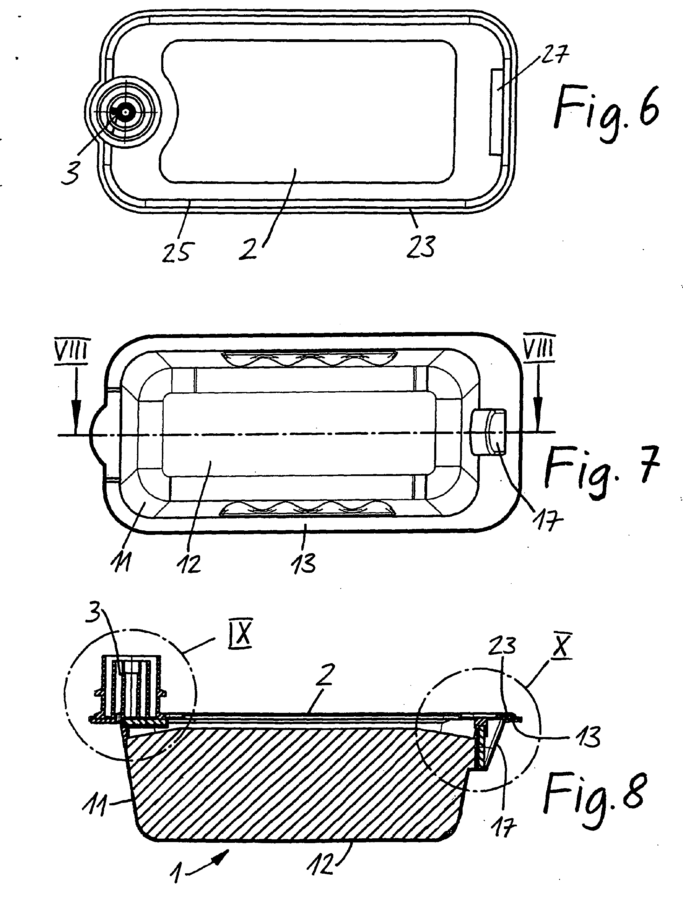

Bei dem in den Fig. 6 bis 10 dargestellten Bicarbonatbehälter der zweiten Ausführungsform sind gleiche bzw. entsprechende Teile mit den gleichen Bezugszeichen versehen. Der Bicarbonatbehälter der zweiten Ausführungsform besteht wiederum aus einem Basisteil 1 und einem darauf aufgesetzten und damit verschweißten Deckelteil 2 und beide Teile sind wiederum im Spritzgussverfahren aus Kunststoff hergestellt. Das Basisteil 1 ist, wie bei der ersten Ausführungsform, als Wanne 11 mit einem Boden 12 und einem oberen Flansch 13 ausgebildet und hat eine im wesentlichen rechteckige Form mit gerundeten Ecken und einer leichten Verjüngung zum Boden 12 hin, kann aber wieder eine beliebige andere Form haben. Der Flansch 13 ist flach und verläuft in der Öffnungsebene der Wanne 11 um die Wannenöffnung herum. Die Oberseite des Flanschs 13 bildet eine Schweißfläche zur Verschweißung mit einer entsprechenden Flanschfläche des Deckelteils 2.

Das Deckelteil 2 hat an seinem Umfang wiederum einen ebenen Flansch 23, der mit dem Flansch 13 des Basisteils 1 zusammenwirkt. In dem Flansch 23 des Deckelteils 2 ist (in Umkehrung der Anordnung bei der ersten Ausführungsform, wo im Flansch 13 des Basisteils 1 eine Rinne 25 eingeformt, die, wenn der Flansch 23 mit dem Flansch 13 verschweißt ist, mit diesem zusammen einen Leitungskanal bildet.

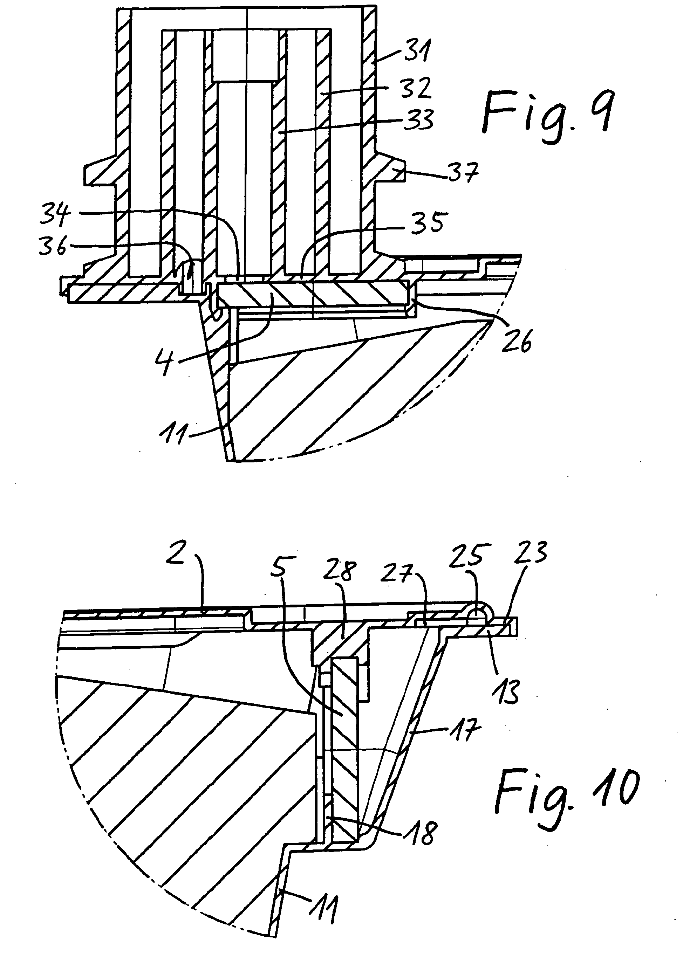

Weiter ist das Deckelteil 2, wie aus den Fig. 6, 8 und 9 ersichtlich, bei der zweiten Ausführungsform ebenso wie bei der ersten Ausführungsform mit einem angeformten Steckverbinder 3 ausgebildet, der, wie die Schnittdarstellung nach Fig. 9 zeigt, eine äußere Führungshülse 31 sowie innerhalb derselben und damit konzentrisch angeordnet zwei koaxiale Leitungsbuchsen 32 und 33 aufweist. Der von der inneren Leitungsbuchse 33 begrenzte innere Leitungskanal des Steckverbinders 3 endigt wiederum in der Ebene des Flanschs 23 in einer zentrischen Öffnung 34, die in das Behälterinnere, also in den Wanneninnenraum der Wanne 11 des Basisteils 1 ausmündet. Jedoch ist diese zentrische Öffnung 34 durch eine Filterscheibe 4, die in einen Haltering 26 an der Unterseite des Deckelteils 2 eingesetzt ist, vom Wanneninnenraum getrennt.

Der zwischen der inneren Leitungsbuchse 33 und der äußeren Leitungsbuchse 32 des Steckverbinders gebildete ringförmige Leitungskanal ist wiederum durch einen Boden 35 abgeschlossen, der eine Durchtrittsöffnung 36 aufweist, die bei der zweiten Ausführungsform direkt mit der im Flansch 23 eingeformten Rinne 25 in Verbindung steht.

Wie bei der ersten Ausführungsform ist auch bei der zweiten Ausführungsform die Wanne 11 des Basisteils 1 an ihrer von dem Steckverbinder 3 entfernten Ende mit einer Ausbauchung 17 versehen. Die im Flansch 23 des Deckelteils 2 gebildete Rinne 25 mündet im Bereich dieser Ausbauchung 17 über eine Übertrittsöffnung 27 in den Innenraum dieser Ausbauchung 17. Wie Fig. 10 in der vergrößerten Schnittdarstellung zeigt, ist der Innenraum der Ausbauchung 17 vom übrigen Innenraum der Wanne 11 durch eine Filterscheibe 5 getrennt, die zwischen an der Wanne 11 bzw. an der Unterseite des Deckelteils 2 angeformten Halteprofilen 18 bzw. 28 gehaltert ist.

Durch die Anordnung der Filterscheiben 4 und 5 in der zweiten Ausfiihrungsform des Bicarbonatbehälters nach der Erfindung kann dieser in beliebiger Weise am Dialysegerät orientiert sein, ohne daß die Gefahr besteht, daß Bicarbonat aus der Basisteilwanne 11 (in Fig. 8 ist die Bicarbonatfüllung schraffiert dargestellt) in unaufgelöster Form aus der Wanne weggeschwemmt wird.

Bei dem erfindungsgemäßen Bicarbonatbehälter (bei beiden dargestellten Ausführungsformen) brauchen also lediglich die beiden im Spritzgussverfahren hergestellten Komponenten Basisteil und Deckelteil durch Verschweißen ihrer Flansche, beispielsweise durch Ultraschallschweißen, miteinander verbunden werden (nachdem zuvor das Bicarbonat in die Wanne 11 des Basisteils eingefüllt worden ist), und es bedarf keines in das Behälterinnere hineinragenden Schlauchs, der gesondert hergestellt und eingesetzt werden müsste und der die Bicarbonatbefüllung behindern oder sich in ungewollter Weise verlagern könnte.

Bei den dargestellten Ausführungsbeispielen ist der Steckverbinder 3 jeweils am Deckelteil 2 angeformt, so dass er beim fertigen Bicarbonatbehälter nach oben vom Deckelteil wegragt. Diese Ausgestaltung ist auch im Hinblick auf einen einfachen Werkzeugbau für die Spritzgusswerkzeuge vorteilhaft, da mit Ausnahme eines Verriegelungselements 37 am Steckverbinder 3 keine Hinterschneidungen und damit keine Schieber notwendig sind.

Jedoch versteht es sich, dass auch andere Anordnungen des Steckverbinders 3 sowohl am Deckelteil als auch am Basisteil und auch andere Ausführungsformen des Steckverbinders (die weitgehend von der Steckverbinderaufnahme des Dialysegeräts abhängt), beispielsweise mit nichtkoaxialen Leitungskanälen, möglich sind.

Bei Ausführungsformen, bei denen der Steckverbinder 3 nicht am Deckelteil, sondern am Basisteil angeordnet ist und auch die Rinne 15 im Flansch 13 des Basisteils gebildet ist, kann es genügen, nur das Basisteil als Kunststoffspritzgussteil auszubilden. Das Deckelteil kann bei einer solchen Ausführungsform dann auf eine einfache Folie reduziert sein, die nach Einfüllen des Bicarbonats in die Wanne des Bassisteils nur noch auf den Flansch um die Wannenöffnung des Basisteils aufgelegt und damit verschweißt wird, um sowohl die Wannenöffnung zu verschließen als auch im Zusammenwirken mit der Rinne im Flansch des Basisteils den Leitungskanal zwischen den voneinander entfernten Wannenenden zu bilden.

## Patentansprüche

1. Bicarbonatbehälter zum Einmalgebrauch in Hämodialysegeräten in der Ausführung als steifer Kunststoffbehälter mit zweikanaligem Steckverbinderanschluß an das Dialysegerät,
bestehend aus einem Basisteil (1) und einem Deckelteil (2) sowie einem vorzugsweise am Deckelteil angeformten zweikanaligen Steckverbinder (3),
wobei das Basisteil (1) eine Wanne (11) zur Aufnahme der Bicarbonatfüllung und einen die Wannenöffnung umgebenden Flansch (13) aufweist und das Deckelteil (2) einen komplementären Flansch (23) aufweist und Basisteil und Deckelteil durch Verschweißen oder dergleichen ihrer Flansche miteinander verbunden sind,
und wobei die beiden Flansche (13, 23) zwischen sich einen vom Behälterinneren getrennten Leitungskanal (15) bilden, der von einem der beiden Steckverbinderkanäle (33, 34) des an einem Ende des Behälters angeordneten Steckverbinders (3) zum anderen Ende des Behälters führt und dort mit dem Behälterinneren in Verbindung steht (16), während der andere Steckverbinderleitungskanal (32, 36) am steckverbinderseitigen Ende des Behälters mit dem Behälterinneren in Verbindung steht (24).

2. Bicarbonatbehälter nach Anspruch 1, wobei der vom einen Steckverbinderleitungskanal (33, 34) zum entgegengesetzten Behälterende führende Leitungskanal (15 oder 25) durch eine im einen (13 oder 23) der beiden Flansche eingeformte und nach Verschweißen der beiden Flansche vom anderen Flansch (23 oder 15) abgedeckte Rinne gebildet ist.

3. Bicarbonatbehälter nach Anspruch 1 oder 2, wobei der Behälter eine etwa rechteckige Grundform hat und der Steckverbinder (3) im Bereich der einen Schmalseite der Rechteckgrundform und die Ausmündung des genannten Leitungskanals (15 oder 25) in das Behälterinnere an der gegenüberliegenden Schmalseite der Rechteckgrundform angeordnet ist.

4. Bicarbonatbehälter nach Anspruch 3, wobei der genannte Leitungskanal (15 oder 25) im wesentlichen entlang der gesamten, bezüglich des Steckverbinders (3) entgegengesetzten Schmalseite der Rechteckgrundform verläuft und über eine Reihe von Durchtrittsöffnungen (16) in das Behälterinnere ausmündet.

5. Bicarbonatbehälter nach einem der Ansprüche 1 bis 4, wobei der Leitungskanal (15 oder 25) an dem vom Steckverbinder (3) entfernten Ende des Behälters in eine beispielsweise als Ausbauchung (10) ausgebildete Erweiterung des Behälterinnenraums mündet, die durch eine Filterscheibe (5) vom übrigen Behälterinnenraum getrennt ist.

6. Bicarbonatbehälter nach einem der Ansprüche 1 bis 5, wobei zwischen der Ausmündung des direkt in das Behälterinnere führenden Steckverbinderleitungskanals und dem Behälterinneren eine Filterscheibe (4) angeordnet ist.

7. Bicarbonatbehälter nach einem der Ansprüche 1 bis 6, wobei das Basisteil (1) und das Deckelteil (2) entlang ihrer aneinanderliegenden Flansche (13, 23) durch Ultraschallverschweißen miteinander verbunden sind.

8. Bicarbonatbehälter nach einem der Ansprüche 1 bis 7, wobei der Steckverbinder (3) als Koaxialstecker mit einer äußeren Führungshülse (31), die vorzugsweise mit einem Verriegelungselement (37) versehen ist, und zwei damit konzentrisch angeordneten koaxialen Leitungsbuchsen (32, 33), die zwei koaxiale Leitungskanäle begrenzen, ausgebildet ist.

9. Bicarbonatbehälter nach einem der Ansprüche 1 bis 8, wobei das Basisteil (1) und das mit dem Steckverbinder (3) ausgebildete Deckelteil (2) jeweils als Kunststoffspritzgussteile ausgebildet sind.

10. Bicarbonatbehälter nach einem der Ansprüche 1 bis 8, wobei das Basisteil mit angeformten Steckverbinder als Kunststoffspritzgussteil ausgebildet ist und das Deckelteil lediglich aus einer auf den Flansch des Basisteils aufgeschweißten oder aufgeklebten Folie besteht.

## Claims

1. Bicarbonate container for single use in haemodialysis apparatuses in the form of a rigid plastics material container with a two-channel plug-in connector for connection to the dialysis apparatus,
consisting of a bottom part (1) and a cover part (2) and also a two-channel plug-in connector (3) which is preferably moulded onto the cover part,
wherein the bottom part (1) has a trough (11) for receiving the bicarbonate filling and a flange (13) surrounding the trough opening and the cover part (2) has a complementary flange (23) and the bottom part and cover part are joined together by welding or the like of their flanges,
and wherein the two flanges (13, 23) form between them a conduit channel (15) which is separated from the interior of the container and leads from one of the two plug-in connector channels (33, 34) of the plug-in connector (3) arranged at one end of the container to the other end of the container and is connected (16) there to the interior of the container, whereas the other plug-in connector conduit channel (32, 36) is connected (24) to the interior of the container at the plug-in connector-side end of the container.

2. Bicarbonate container according to claim 1, wherein the conduit channel (15 or 25) leading from a plug-in connector conduit channel (33, 34) to the opposite container end is formed by a groove which is formed into one (13 or 23) of the two flanges and is covered by the other flange (23 or 15) after welding of the two flanges.

3. Bicarbonate container according to claim 1 or 2, wherein the container has an approximately rectangular basic shape and the plug-in connector (3) is arranged in the region of one narrow side of the rectangular basic shape and the point at which the aforementioned conduit channel (15 or 25) emerges into the interior of the container is arranged at the opposing narrow side of the rectangular basic shape.

4. Bicarbonate container according to claim 3, wherein the aforementioned conduit channel (15 or 25) runs substantially along the entire narrow side, opposing the plug-in connector (3), of the rectangular basic shape and emerges into the interior of the container via a series of through-openings (16).

5. Bicarbonate container according to one of claims 1 to 4, wherein the conduit channel (15 or 25) emerges at the end of the container that is remote from the plug-in connector (3) into an extension, formed for example as a bulging (10), of the interior of the container, which extension is separated from the remainder of the interior of the container by a filter disc (5).

6. Bicarbonate container according to one of claims 1 to 5, wherein a filter disc (4) is arranged between the emerging of the plug-in connector conduit channel leading directly into the interior of the container and the interior of the container.

7. Bicarbonate container according to one of claims 1 to 6, wherein the bottom part (1) and the cover part (2) are joined together along their adjoining flanges (13, 23) by ultrasonic welding.

8. Bicarbonate container according to one of claims 1 to 7, wherein the plug-in connector (3) is embodied as a coaxial plug with an outer guide sleeve (31), which is preferably provided with a locking element (37), and two coaxial conductor bushings (32, 33) which are arranged concentrically therewith and delimit two coaxial conduit channels.

9. Bicarbonate container according to one of claims 1 to 8, wherein the bottom part (1) and the cover part (2) which is embodied with the plug-in connector (3) are each embodied as plastics material injection-moulded parts.

10. Bicarbonate container according to one of claims 1 to 8, wherein the bottom part with the moulded-on plug-in connector is embodied as a plastics material injection-moulded part and the cover part consists merely of a foil which is welded or adhesively bonded onto the flange of the bottom part.

## Revendications

1. Récipient à bicarbonate à usage unique dans des appareils d'hémodialyse réalisé sous la forme de récipient en matière plastique rigide avec raccordement à l'appareil de dialyse par connecteur enfichable à deux conduits,
composé d'une partie base (1) et d'une partie couvercle (2), ainsi que d'un connecteur enfichable (3) à deux conduits formé de préférence sur la partie couvercle,
la partie base (1) présentant une cuve (11) destinée à recevoir la charge de bicarbonate et une bride (13) entourant l'ouverture de la cuve et la partie couvercle (2) présentant une bride (23) complémentaire et la partie base et la partie couvercle étant reliées entre elles par soudage ou similaire de leurs brides,
et les deux brides (13, 23) formant entre elles un conduit (15) séparé de l'intérieur du récipient, qui conduit de l'un des deux conduits de connecteur enfichable (33, 34) du connecteur enfichable (3) disposé à une extrémité du récipient à l'autre extrémité du récipient et, là, est en liaison (16) avec l'intérieur du récipient, tandis que l'autre conduit de connecteur enfichable (32, 36) est en liaison (24) avec l'intérieur du récipient à l'extrémité du récipient située du côté connecteur enfichable.

2. Récipient à bicarbonate selon la revendication 1, dans lequel le conduit (15 ou 25) conduisant d'un conduit de connecteur enfichable (33, 34) à l'extrémité de récipient opposée est formé par une rainure moulée dans l'une (13 ou 23) des deux brides et recouverte par l'autre bride (23 ou 15) après soudage des deux brides.

3. Récipient à bicarbonate selon la revendication 1 ou 2, dans lequel le récipient a une forme de base approximativement rectangulaire et le connecteur enfichable (3) est disposé dans la zone d'un petit côté de la forme de base rectangulaire et l'embouchure du conduit cité (15 ou 25) vers l'intérieur du récipient est disposée sur le petit côté opposé de la forme de base rectangulaire.

4. Récipient à bicarbonate selon la revendication 3, dans lequel le conduit cité (15 ou 25) s'étend essentiellement le long de tout le petit côté de la forme de base rectangulaire opposé au connecteur enfichable (3) et débouche dans l'intérieur du récipient par l'intermédiaire d'une série d'ouvertures de passage (16).

5. Récipient à bicarbonate selon l'une des revendications 1 à 4, dans lequel le conduit (15 ou 25) débouche à l'extrémité du récipient éloignée du connecteur enfichable (3) dans un élargissement de l'espace intérieur du récipient réalisé sous la forme d'un évasement (10), par exemple, qui est séparé du reste de l'espace intérieur du récipient par un disque filtrant (5).

6. Récipient à bicarbonate selon l'une des revendications 1 à 5, dans lequel un disque filtrant (4) est disposé entre l'embouchure du conduit de connecteur enfichable conduisant directement à l'intérieur du récipient et l'intérieur du récipient.

7. Récipient à bicarbonate selon l'une des revendications 1 à 6, dans lequel la partie base (1) et la partie couvercle (2) sont reliées entre elles le long de leurs brides (13, 23) adjacentes par soudage par ultrasons.

8. Récipient à bicarbonate selon l'une des revendications 1 à 7, dans lequel le connecteur enfichable (3) est réalisé sous la forme de fiche coaxiale avec une boîte de guidage extérieure (31), qui est munie de préférence d'un élément de verrouillage (37), et deux douilles coaxiales (32, 33) disposées de manière concentrique à celle-ci, qui délimitent deux conduits coaxiaux.

9. Récipient à bicarbonate selon l'une des revendications 1 à 8, dans lequel la partie base (1) et la partie couvercle (2) formée avec le connecteur enfichable (3) sont réalisées chacune sous la forme de pièces en matière plastique moulées par injection.

10. Récipient à bicarbonate selon l'une des revendications 1 à 8, dans lequel la partie base avec connecteur enfichable formé dessus est réalisée sous la forme de pièce en matière plastique moulée par injection et la partie couvercle est constituée simplement d'un film soudé ou collé sur la bride de la partie base.
